# EUROPEAN PATENT APPLICATION

(11) **EP 3 048 577 A1**
(43) Date of publication of application: **27.07.2016**
(21) Application number: 13893785.9
(22) Date of filing: 17.09.2013
(51) Int. Cl.: G06Q 50/22

(54) **COLONY EXAMINATION PROGRAM, COLONY EXAMINATION DEVICE, AND COLONY EXAMINATION METHOD**

(71) Applicant: Fujitsu Limited, Kawasaki-shi, Kanagawa 211-8588 (JP)
(72) Inventor: HIDAKA, Kouichi, Kawasaki-shi Kanagawa 211-8588 (JP); NAITO, Hirohisa, Kawasaki-shi Kanagawa 211-8588 (JP); HAYASHI, Mimiko, Kawasaki-shi Kanagawa 211-8588 (JP); SAGA, Susumu, Kawasaki-shi Kanagawa 211-8588 (JP); MIYAJIMA, Sachiko, Kawasaki-shi Kanagawa 211-8588 (JP); KAWANO, Kiyoshi, Kawasaki-shi Kanagawa 211-8588 (JP); MIYAZAKI, Akira, Kawasaki-shi Kanagawa 211-8588 (JP)
(74) Representative: Hoffmann Eitle
(86) International application number: PCT/JP2013/075059
(87) International publication number: WO 2015/040678

(57) **Abstract**

A colony examination device (100) obtains, for each of a plurality of specimens containing colonies of bacteria, a piece of information used for identifying the specimen. Further, the colony examination device (100) arranges the pieces of information identifying the specimens, based on a time period a product represented by each of the specimens takes to arrive at a shipment delivery destination from a facility that produced the specimen, the time period being indicated in each of the obtained pieces of information identifying the specimens. Further, the colony examination device (100) displays the arranged pieces of information identifying the specimens, in a list.

## Description

### [Technical Field]

The present invention is related to a colony examination computer program, a colony examination device, and a colony examination method.

### [Background Art]

When products are shipped, sanitary examinations are performed thereon. For instance, in an example where food items are the products, a part of each of the food items is collected as a specimen, the specimen is either smeared onto or poured into a culture medium prepared in a Petri dish, so that the bacteria contained in the specimen are cultured in the Petri dish for a predetermined time period while being kept at a suitable temperature. After that, a sanitary examination is performed by counting the colonies of bacteria in the Petri dish when the culturing process is completed.

When the sanitary examination is performed in the manner described above, information (e.g., specimen numbers) used for identifying different specimens are arranged in a list, according to the order in which the specimens were received. By using the list, an examiner performs the sanitary examinations according to the order in which the specimens are arranged in the list. A person in charge of the examinations judges whether each of the examination results is appropriate or not, according to the order the results are arranged in the list, so as to determine a final result as to whether each of the specimens has passed or failed.

### [Citation List]

### [Patent Citation]

Patent Document 1: Japanese Laid-open Patent Publication No. 09-187270
Patent Document 2: Japanese Laid-open Patent Publication No. 2007-094513

### [Summary of Invention]

### [Technical Problem]

When the technique described above is used, however, there may be some situations where it is not possible to appropriately set the order in which the specimens are examined.

More specifically, a disadvantageous situation may arise when the specimens are arranged in the list according to the order in which the specimens were received, as described above. For example, when sanitary examinations are performed after shipping products, there may be some situations where, while products that have already been delivered to the destination are being neglected, the sanitary examinations are first performed on other products that have not yet been delivered to the destination. In those circumstances, a disadvantageous situation may arise where a delay occurs in the sanitary examination to be performed on the neglected products, which also causes a delay in storefront shelving of the products delivered from a shipping facility or in secondary processing to be applied to the products delivered from the shipping facility. In another example, when sanitary examinations are performed before shipping products, there may be some situations where, while products having a long transport time period to the destination are being neglected, the sanitary examinations are first performed on other products having a short transport institution. In these circumstances also, a disadvantageous situation may arise where the arrival of the neglected products at the destination get behind schedule and where it is impossible to perform storefront shelving and/or secondary processing as scheduled at the delivery destination.

In the explanation above, the examples are explained in which the sanitary examinations are performed according to the order in which the specimens are arranged in the list. However, a similarly disadvantageous situation may arise even when the specimens are examined according to the order in which the specimens are arranged in the list, because the sanitary examinations are performed according to the order in which image taking processes on the specimens are completed.

An object of at least one aspect of the present invention is to provide a colony examination computer program, a colony examination device, and a colony examination method with which it is possible to appropriately set the order in which the specimens are examined or in which the images of the specimens are taken.

### [Solution to Problem]

According to a first aspect, a colony examination computer program causes a computer to execute a process including obtaining, for each of a plurality of specimens from which colonies of bacteria are cultured, a piece of information used for identifying the specimen. Furthermore, the process includes arranging the pieces of information identifying the specimens, based on a time period a product represented by each of the specimens takes to arrive at a shipment delivery destination from a facility that produced the specimen, the time period being indicated in each of the obtained pieces of information identifying the specimens. Furthermore, the process includes displaying the arranged pieces of information identifying the specimens, in a list.

### [Advantageous Effects of Invention]

It is possible to appropriately set the order in which the specimens are examined or in which the images of the specimens are taken.

### [Brief Description of Drawings]

FIG. 1 is a diagram illustrating a configuration of the entirety of a system according to a first embodiment.
FIG. 2 is a functional block diagram illustrating a configuration of a colony examination device according to the first embodiment.
FIG. 3 is a table illustrating an example of a data structure of an examination database (DB).
FIG. 4 is a table illustrating a first example of a specimen list created by an arranging unit.
FIG. 5 is a drawing of an example of a display of a result judgment window.
FIG. 6 is a table illustrating a second example of a result judgment list.
FIG. 7 is a table illustrating a third example of the result judgment list.
FIG. 8 is a flowchart of an example of a processing operation up to when the colony examination device displays a result judgment list.
FIG. 9 is a flowchart of an example of a result judging process.
FIG. 10 is a table illustrating a second example of the specimen list created by the arranging unit.
FIG. 11 is a table illustrating a fourth example of the result judgment list.
FIG. 12 is a table illustrating a fifth example of the result judgment list.
FIG. 13 is a table illustrating a sixth example of the result judgment list.
FIG. 14 is a flowchart of an example of a processing operation up to when the colony examination device displays a result judgment list.
FIG. 15 is a table illustrating a specimen list corresponding to when there are multiple values of transport time periods with respect to a single lot.
FIG. 16 is a table illustrating a first example of a specimen list rearranged by the arranging unit when there are multiple values of transport time periods with respect to a single lot.
FIG. 17 is a table illustrating a second example of the specimen list rearranged by the arranging unit when there are multiple values of transport time periods with respect to a single lot.
FIG. 18 is a table illustrating an example of a specimen list containing expiration dates/times as items thereof.
FIG. 19 is a table illustrating an example of a specimen list containing expiration dates/times as items thereof and having been rearranged by the arranging unit.
FIG. 20 is a diagram illustrating a hardware configuration of a computer related to a colony examination device.

### [Embodiments for Carrying Out the Invention]

Exemplary embodiments of a colony examination computer program, a colony examination device, and a colony examination method disclosed herein will be explained in detail below, with reference to the accompanying drawings. The present invention is not limited to the exemplary embodiments. It is acceptable to combine any of the embodiments together as appropriate, as long as no conflict arises among the contents of the processing.

### <First Embodiment>

### <A configuration of the entirety of a system>

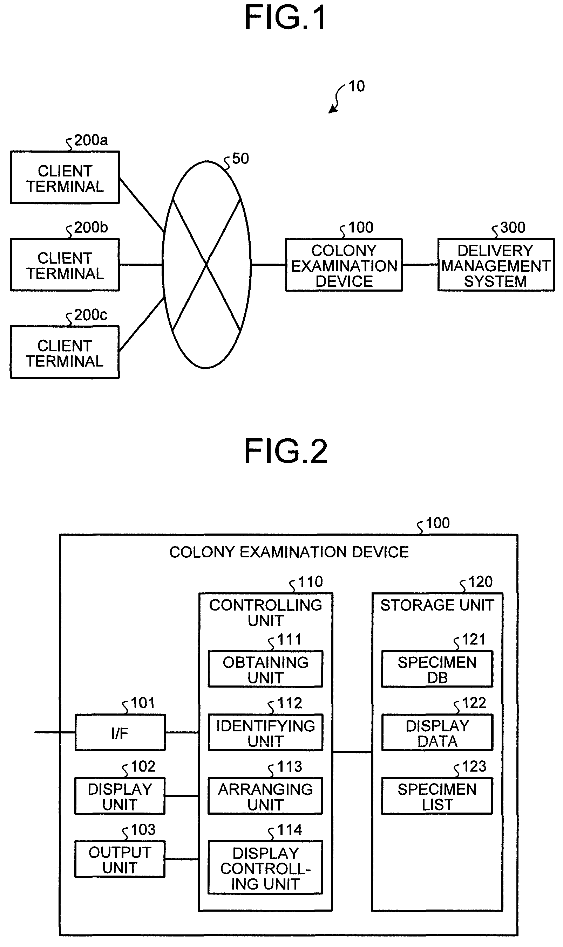

Next, a configuration of the entirety of a system according to a first embodiment will be explained. FIG. 1 is a diagram illustrating a configuration of the entirety of the system according to the first embodiment. As illustrated in FIG. 1, a system 10 includes client terminals 200a to 200c, a delivery management system 300, a network 50, and a colony examination device 100.

The client terminals 200a to 200c are connected to the colony examination device 100 via the network 50. Further, the colony examination device 100 is connected to the delivery management system 300. The colony examination device 100 notifies the delivery management system 300 of examination results of products. Each of the client terminals 200 accesses the colony examination device 100 via the network 50 and inputs the examination results to the colony examination device 100. The delivery management system 300 obtains the examination results from the colony examination device 100. In the following sections, a window used for inputting the examination results will be referred to as an examination window.

### <Sanitary examinations>

Next, a procedure performed by an examiner as a preparation for a sanitary examination will be explained by using a specific example. For example, at first, the examiner collects a part of a food item as a specimen and assigns a specimen number to the specimen. Subsequently, when using a pour plate method, for example, the examiner puts the collected specimen into Petri dishes together with a dissolved culture medium, in correspondence with various types of examination items such as general viable bacteria, Escherichia coli (hereinafter "E. coli"), and the like. Also, in that situation, the examiner writes the specimen numbers on the lids of the Petri dishes, so as to be able to distinguish the Petri dishes from one another. Subsequently, the examiner allows the bacteria in the Petri dishes to be cultured, by keeping the Petri dishes for one to two days at a temperature suitable for growth of the bacteria. After the culturing process is completed, the examiner counts, according to a specimen list, the number of colonies in each of the Petri dishes either visually or by using a colony counter.

### <A functional configuration of the colony examination device>

An example of a functional configuration of the colony examination device 100 according to the first embodiment will be explained. FIG. 2 is a functional block diagram of a configuration of the colony examination device according to the first embodiment. As illustrated in FIG. 2, the colony examination device 100 includes an interface (I/F) 101, a display unit 102, an output unit 103, a controlling unit 110, and a storage unit 120. The I/F 101 is a communication interface that is connected to the network 50 and is configured to transmit data to each of the terminal devices 200 via the network 50. The display unit 102 displays the examination window of the colony examination device 100 on a monitor. Further, the output unit 103 outputs a specimen list 123 created by the colony examination device 100.

### <Constituent elements of the storage unit>

The storage unit 120 stores therein an examination database (DB) 121, display data 122, and the specimen list 123. The storage unit 120 corresponds to, for example, a semiconductor memory device such as a Random Access Memory (RAM), a Read-Only Memory (ROM), or a flash memory, or a storage device such as a hard disk or an optical disk.

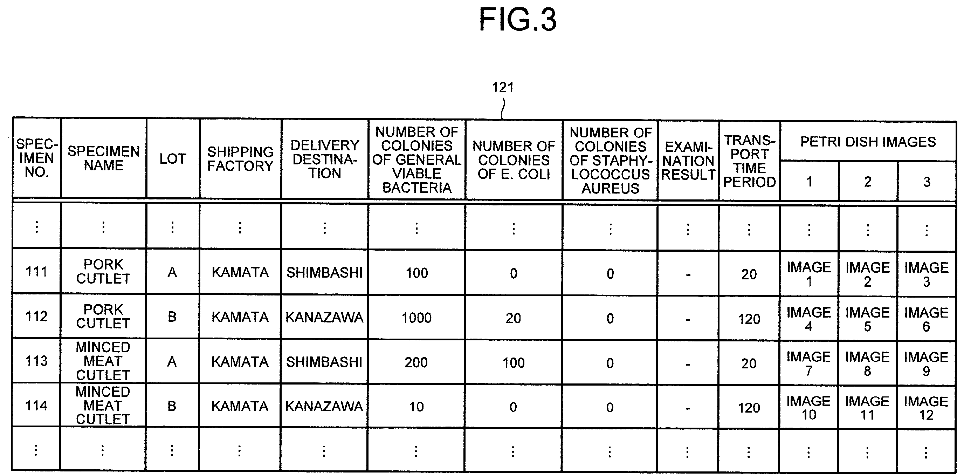

The examination DB 121 is a database for storing therein examination items, an examination result, a transport time period, and the like, for each of the specimens, so as to be kept in correspondence with one another. FIG. 3 is a table illustrating an example of a data structure of the examination DB. As illustrated in FIG. 3, the examination DB 121 stores the following items that are kept in correspondence with one another: a specimen number, a specimen name, a lot, a shipping factory, a delivery destination, the number of colonies of general viable bacteria, the number of colonies of E. coli, the number of colonies of Staphylococcus aureus, an examination result, a transport time period, Petri dish image 1, Petri dish image 2, and Petri dish image 3.

The "specimen number (No.)" is a number that is uniquely assigned to each of the specimens. The specimen number may uniquely be assigned to each of the specimens for each examination reception date. The "specimen name" indicates the product to be examined. The "lot" is a symbol that is uniquely assigned to products to be examined that are the same as one another. The "shipping factory" indicates the location of the factory that ships the product. The "delivery destination" indicates the location to which the product is to be delivered. The "number of colonies of general viable bacteria" indicates the number of colonies of general viable bacterial contained in the specimen. The "number of colonies of E. coli" indicates the number of colonies of E. coli contained in the specimen. The "number of colonies of Staphylococcus aureus" indicates the number of colonies of Staphylococcus aureus contained in the specimen. The "examination result" indicates a result of sanitary examinations performed on each of the products. For example, if the results of the sanitary examinations are normal, the "examination result" is indicated as "OK". On the contrary, if the results of the sanitary examinations are abnormal, the "examination result" is indicated as "not OK". When the sanitary examination has not yet been performed, the "examination result" is indicated as "-". The "transport time period" indicates the time period it takes to transport the product from the shipping factory to the delivery destination. "Petri dish image 1" is a Petri dish image for examining general viable bacteria. "Petri dish image 2" is a Petri dish image for examining E. coli. "Petri dish image 3" is a Petri dish image for examining Staphylococcus aureus.

The display data 122 is data containing the images taken of the Petri dishes. In the following sections, an image taken of a Petri dish will be referred to as a "Petri dish image". Further, the display data 122 keeps the Petri dish images in correspondence with the specimen number of the corresponding specimen. In this situation, the file format of the display data 122 may be, for example, a Graphics Interchange Format (GIF) file, a Joint Photographic Experts Group (JPEG) file, a bitmap (BMP) file, or the like.

The specimen list 123 contains data arranged in the rows obtained from the examination DB 121. The configuration of the examination list 123 may be the same as that of the examination DB 121. The specimen list 123 is obtained by extracting, from the specimen DB 121, one or more rows corresponding to the specimens that are soon to be examined. Accordingly, the specimen list 123 does not contain any data of finished examinations. In the following sections, the data arranged in each of the rows of the specimen DB 121 will be referred to as "specimen data".

### <Constituent elements of the controlling unit>

The controlling unit 110 includes an obtaining unit 111, an identifying unit 112, an arranging unit 113, and a display controlling unit 114. Functions of the controlling unit 110 may be realized by, for example, causing a Central Processing Unit (CPU) to execute a predetermined computer program. Alternatively, functions of the controlling unit 110 may be realized by using, for example, an integrated circuit such as an Application Specific Integrated Circuit (ASIC) or a Field Programmable Gate Array (FPGA).

In the first embodiment, a mode in which sanitary examinations are performed on products after shipping will be explained. For example, products shipped from a facility such as a shipping factory include products having a short time period before the expiration dates/times such as perishable food products. When transporting such products, the facility may ship the products before performing examinations thereon, because shipping the products after performing the examinations may degrade the freshness of the products. Further, the facility notifies the product delivery destination of examination results after the products are shipped. A process performed by the constituent elements of the controlling unit 110 in that situation will be explained below.

For each of a plurality of Petri dishes containing colonies of bacteria, the obtaining unit 111 obtains a piece of information used for identifying the specimen. For example, the obtaining unit 111 obtains pieces of data corresponding to the specimen numbers 111 to 114 to be examined, from the specimen DB 121 illustrated in FIG. 3.

The identifying unit 112 identifies the specimen from which colonies of bacteria have been cultured in each of the plurality of Petri dishes. The identifying unit 112 identifies each of the specimens by using the specimen numbers of the pieces of specimen data obtained from the specimen DB 121.

The arranging unit 113 arranges the pieces of information identifying the obtained specimens, based on the time periods it takes for the products represented by the specimens to arrive at the shipment delivery destination from the facility that produced the identified specimens. In other words, the arranging unit 113 outputs a specimen list in which the pieces of specimen data obtained by the obtaining unit 111 have been rearranged in ascending order of the transport time periods.

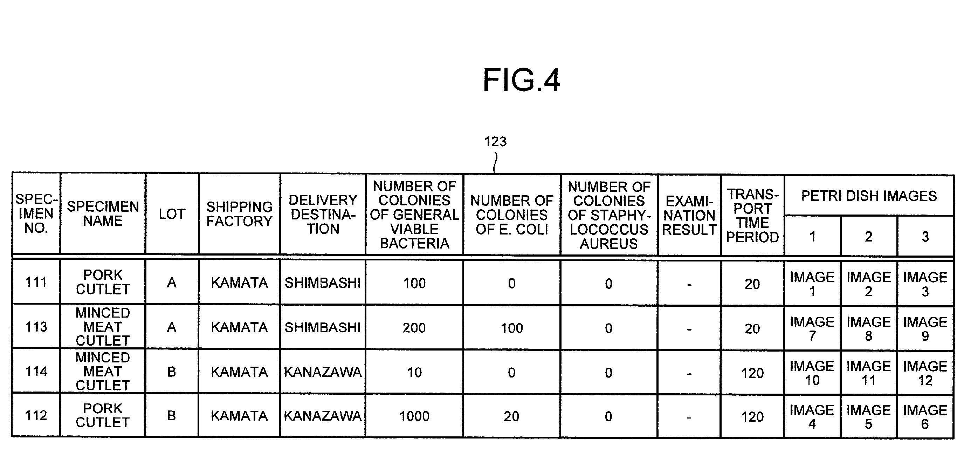

Processes performed by the arranging unit 113 and the display controlling unit 114 when the sanitary examinations are performed on products after shipping will be explained with reference to FIGS. 4 to 8. FIG. 4 is a table illustrating a first example of the specimen list in which the pieces of specimen data have been rearranged by the arranging unit. The arranging unit 113 obtains the transport time periods "20", "120", "20", and "120" from the pieces of specimen data corresponding to the specimen numbers "111", "112", "113", and "114". Subsequently, the arranging unit 113 rearranges the pieces of specimen data into the ascending order of the transport time periods. In other words, the arranging unit 113 rearranges the pieces of specimen data, so that the specimen numbers are in the order of "111", "113" "114", and "112", for example. After that, the arranging unit 113 stores the rearranged specimen list 123 into the storage unit 120. In other words, the arranging unit 113 outputs the specimen list in which the specimen numbers obtained by the obtaining unit 111 have been rearranged into the ascending order of the transport time periods. In this situation, because the transport time periods of specimens Nos. "111" and "113" and the transport time periods of specimens Nos. "112" and "114" are equal to each other, the arranging unit 113 may arrange the two specimens having mutually-the-same transport time period in the opposite order in the specimen list.

The display controlling unit 114 displays a result judgment window 400 used by a person who is in charge of the examination to input an examination result. The display controlling unit 114 displays a result judgment list 401 in the result judgment window 400. The result judgment list 401 is a list indicating the number of colonies of bacteria corresponding to any of the examination items and judgment results.

The culture time period for the examined bacteria varies for each of the examination items. For this reason, the display controlling unit 114 may display result judgment lists 401 in ascending order of the culture time periods of the examined bacteria. For example, when the examination items include examinations for the number of colonies of Staphylococcus aureus, E. coli, and general viable bacteria, the display controlling unit 114 performs the following process: When the examination is completed on Staphylococcus aureus, which has the shortest time period for the bacteria to finish being cultured, the display controlling unit 114 causes the number of colonies to be displayed in a result judgment list 401. Subsequently, after the examination on Staphylococcus aureus is completed, the display controlling unit 114 causes the number of colonies of E. coli, which has the second shortest time period for the bacteria to finish being cultured, to be displayed. Subsequently, after the examination on E. coli is completed, the display controlling unit 114 causes the number of colonies of general viable bacteria, which has the longest time period for the bacteria to finish being cultured, to be displayed.

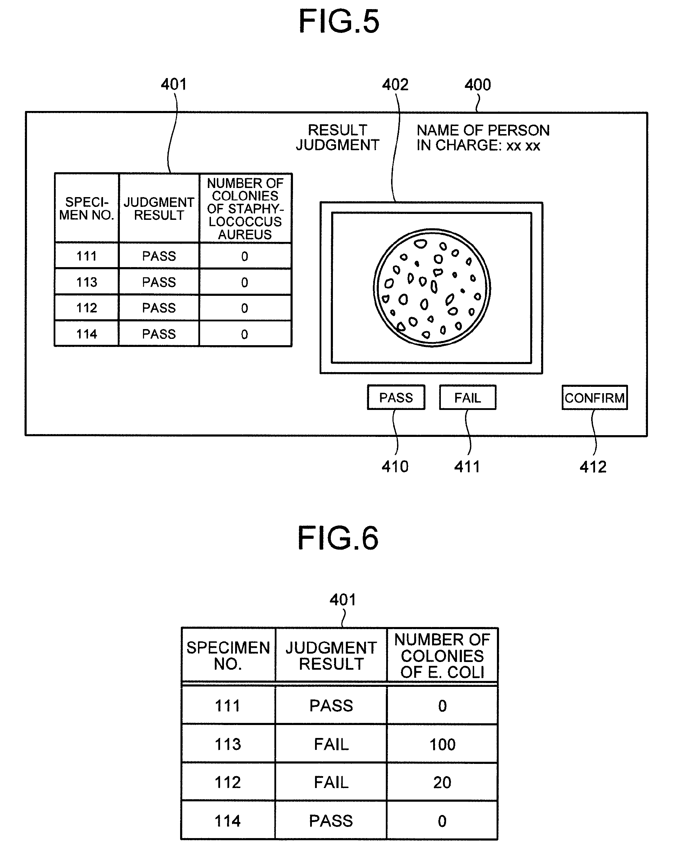

FIG. 5 is a drawing of an example of the display of the result judgment window. The result judgment window 400 includes the result judgment list 401, an image display area 402, a PASS (i.e., within the prescribed range) button 410, a FAIL (i.e., outside the prescribed range) button 411, and a CONFIRM button 412. The result judgment list 401 indicates judgment results and the numbers of colonies of bacteria corresponding to any of the examination items. The image display area 402 is an area used for displaying a Petri dish image corresponding to the specimen number selected in the result judgment list 401. The PASS button 410 is a button pressed when the number of colonies is normal. The FAIL button 411 is a button pressed when the number of colonies is abnormal. The CONFIRM button 412 is a button pressed to confirm the selection made on either the PASS button 410 or the FAIL button 411.

Next, the items contained in the result judgment list 401 will be explained. The result judgment list 401 indicates the specimen numbers, judgment results, and the number of colonies of Staphylococcus aureus that are kept in correspondence with one another. Each of the "specimen numbers (No.)" is a number uniquely assigned to a specimen, and the numbers are the same as those used in the specimen DB 121. Each of the "judgment results" indicates whether the number of colonies of Staphylococcus aureus is within the prescribed range (PASSED) or outside the prescribed range (FAILED). When the judging process has not yet been performed, the "judgment result" is indicated as "-". Each of the entries in the "Number of colonies of Staphylococcus aureus" column indicates the number colonies of Staphylococcus aureus contained in the specimen. The "Number of colonies of Staphylococcus aureus" column will be indicated as "Number of colonies of E. coli" when the judging process is performed on the number of colonies of E. coli and will be indicated as "Number of colonies of general viable bacteria" when the judging process is performed on the number of colonies of general viable bacteria.

Processes performed by the arranging unit 113 and the display controlling unit 114 will be explained, by using a specific example. First, the display controlling unit 114 displays the result judgment list 401 for Staphylococcus aureus in the result judgment window 400. In this situation, in the result judgment list 401, the number of colonies of Staphylococcus aureus has been input in the "Number of colonies of Staphylococcus aureus" column, but the "judgment result" column is indicated as "-".

Subsequently, the display controlling unit 114 inputs judgment results in the "judgment result" column of the result judgment window 400 according to the judgment results that correspond to the pieces of specimen data and are input by the examiner. For example, when the PASS button 410 is pressed in the result judgment window 400, the display controlling unit 114 stores, into the storage unit 120, judgment data including information indicating that the judgment result says PASSED. On the contrary, when the FAIL button 411 is pressed in the result judgment window 400, the display controlling unit 114 stores, into the storage unit 120, judgment data including information indicating that the judgment result says FAILED. In other words, the judgment data represents the piece of data in each of the rows of the result judgment list 401. In an example, the display controlling unit 114 may display the Petri dish image for the next row in the next result judgment list 401 when either the PASS button 410 or the FAIL button 411 is pressed in the result judgment window 400. Alternatively, the display controlling unit 114 may display the next Petri dish image when one of the rows in the result judgment list is clicked.

For example, while specimen No. "111" is selected in the result judgment window 400, when the examiner presses the PASS button 410, the display controlling unit 114 displays the judgment result as "PASSED". Subsequently, while specimen No. "113" is selected in the result judgment window 400, when the examiner presses the PASS button 410, the display controlling unit 114 displays the judgment result as "PASSED". After that, while specimen No. "112" is selected in the result judgment window 400, when the examiner presses the PASS button 410, the display controlling unit 114 displays the judgment result as "PASSED". Further, while specimen No. "114" is selected in the result judgment window 400, when the examiner presses the PASS button 410, the display controlling unit 114 displays the judgment result as "PASSED".

At this point in time, the result judgment list 401 displays the number of colonies of Staphylococcus aureus for each of the specimens. According to the order in which the examination is completed, the result judgment list 401 displays the numbers of colonies in the order of Staphylococcus aureus, E. coli, and general viable bacteria. When the FAIL button 411 is pressed, and subsequently, the CONFIRM button 412 is pressed in the result judgment window 400 displaying the examination results, the display controlling unit 114 stores, into the storage unit 120, judgment data indicating that the judgment result says FAILED. Subsequently, the arranging unit 113 rearranges the corresponding piece of judgment data into an upper position, based on the judgment result indicating that the judgment result says FAILED. After that, when proceeding to the next examination item, the display controlling unit 114 reads the rearranged pieces of judgment data and outputs the read data to the result judgment window 400.

For example, as illustrated in FIG. 5, as for the numbers of colonies of Staphylococcus aureus, because all the pieces of specimen data are indicated as "0", "PASSED" is input for each of all the judgment results. The display controlling unit 114 stores the judgment results into the storage unit 120 as pieces of judgment data. Further, because all the judgment results are indicated as "PASSED", the arranging unit 113 does not rearrange the pieces of specimen data.

After the judging process on Staphylococcus aureus is completed, the display controlling unit 114 displays the result judgment list 401 for E. coli, based on the stored judgment data. In this situation, in the result judgment list 401, although the numbers of colonies of E. coli have been input in the "Number of colonies of E. coli" column, the "judgment result" column is indicated as "-", because the judgment results have not yet been input.

Subsequently, for example, while specimen No. "111" is selected in the result judgment window 400, when the examiner presses the PASS button 410, the display controlling unit 114 displays the judgment result as "PASSED". Subsequently, while specimen No. "113" is selected in the result judgment window 400, when the examiner presses the FAIL button 411, the display controlling unit 114 displays the judgment result as "FAILED". After that, while specimen No. "112" is selected in the result judgment window 400, when the examiner presses the FAIL button 411, the display controlling unit 114 displays the judgment result as "FAILED". Further, while specimen No. "114" is selected in the result judgment window 400, when the examiner presses the PASS button 410, the display controlling unit 114 displays the judgment result as "PASSED".

FIG. 6 is a table illustrating a second example of the result judgment list. The result judgment list 401 in FIG. 6 illustrates only the section extracting the result judgment list 401 displayed in the result judgment window 400. At this point in time, the result judgment list 401 indicates the number of colonies of E. coli for each of the specimens. In the result judgment list 401 in FIG. 6, the judgment results for specimen No. 112 and specimen No. 113 are indicated as "FAILED". The display controlling unit 114 stores the judgment results into the storage unit 120 as pieces of judgment data. Subsequently, the arranging unit 113 rearranges the pieces of specimen data so that specimen No. 112 having the shorter transport time period is displayed in the first row and so that specimen No. 113, which is ranked next according to the ascending order of the transport time periods, is displayed in the second row.

After the judging process on E. coli is completed, the display controlling unit 114 displays the result judgment list 401 for general viable bacteria, based on the stored judgment data. In this situation, in the result judgment list 401, although the numbers of colonies of general viable bacteria have been input in the "Number of colonies of general viable bacteria" column, the "judgment result" column is indicated as "-", because the judgment results have not yet been input. Similarly to the examples of the examinations for Staphylococcus aureus and E. coli, the display controlling unit 114 stores pieces of judgment data into the storage unit 120, based on the judgment results.

After that, for example, while specimen No. "113" is selected in the result judgment window 400, when the examiner presses the FAIL button 411, the display controlling unit 114 displays the judgment result as "FAILED". Subsequently, while specimen No. "112" is selected in the result judgment window 400, when the examiner presses the FAIL button 411, the display controlling unit 114 displays the judgment result as "FAILED". After that, while specimen No. "111" is selected in the result judgment window 400, when the examiner presses the PASS button 410, the display controlling unit 114 displays the judgment result as "PASSED". Further, while specimen No. "114" is selected in the result judgment window 400, when the examiner presses the PASS button 410, the display controlling unit 114 displays the judgment result as "PASSED".

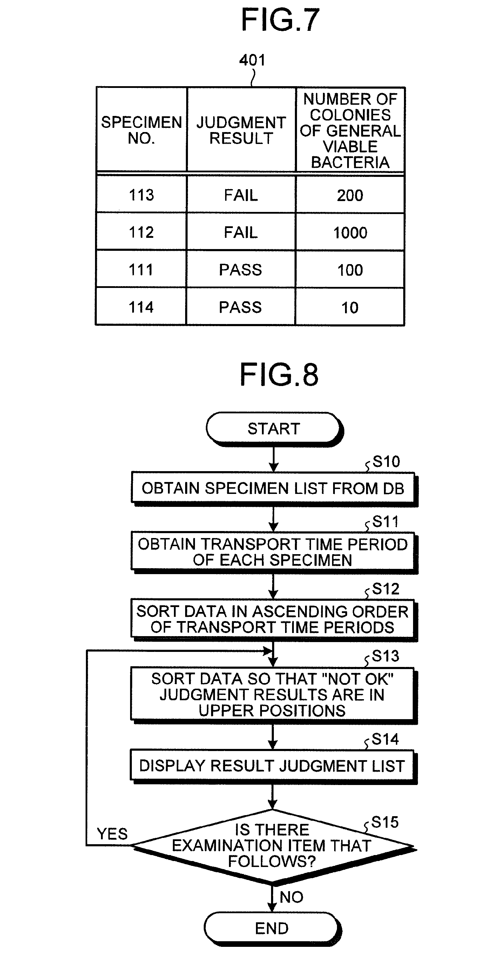

FIG. 7 is a table illustrating a third example of the result judgment list. The result judgment list 401 in FIG. 7 illustrates only the section extracting the result judgment list 401 from the result judgment window 400. The display controlling unit 114 displays the pieces of specimen data in the result judgment list 401, according to the order rearranged by the arranging unit 113.

After that, the colony examination device 100 updates the "examination result" column of the specimen list 123, based on the pieces of judgment data for each of the examination items. For each of the examination items, if at least one of the judgment results is indicated as "FAILED", the colony examination device 100 inputs "not OK" in the "examination result" column of the specimen list 123.

For example, the colony examination device 100 inputs "OK" in the examination result column corresponding to specimen No. 111. Further, the colony examination device 100 inputs "not OK" in the examination result column corresponding to specimen No. 112. Also, the colony examination device 100 inputs "not OK" in the examination result column corresponding to specimen No. 113. In addition, the colony examination device 100 inputs "OK" in the examination result column of the specimen list 123 corresponding to specimen No. 114.

Further, when each of the rows in the result judgment list 401 is clicked in the result judgment window 400, the display controlling unit 114 displays the Petri dish image corresponding to the specimen number in the clicked row. Alternatively, when the CONFIRM button 412 has been pressed to confirm a judgment result, the display controlling unit 114 may automatically switch the displayed Petri dish image to the next Petri dish image.

### <A flow in a process performed by the colony examination device>

Next, a flow in a process performed by the colony examination device 100 will be explained. FIG. 8 is a flowchart of an example of a processing operation up to when the colony examination device displays a result judgment list. First, the obtaining unit 111 obtains a specimen list 123 from the specimen DB 121 (step S10). For example, the specimen list 123 is obtained by extracting pieces of data in such rows that correspond to the specimens to be examined, from the specimen DB 121. Accordingly, the specimen list 123 does not contain any data of finished examinations. Subsequently, the identifying unit 112 identifies the specimens based on the specimen numbers of the pieces of specimen data obtained from the specimen DB 121.

After that, the arranging unit 113 obtains the transport time period of each of the specimens, from the pieces of specimen data in the specimen list (step S11). Subsequently, the arranging unit 113 rearranges the pieces of specimen data in the ascending order of the transport time periods in the specimen list (step S12). After that, the arranging unit 113 rearranges one or more pieces of specimen data of which the judgment results in the specimen list are indicated as "not OK" into upper positions (step S13). The arranging unit 113 stores the rearranged specimen list into the storage unit 120.

Subsequently, the display controlling unit 114 reads the rearranged specimen list 123 from the storage unit 120, and further, copies the "specimen numbers" and the numbers of colonies of bacteria related to the examination item for which the culturing processes have been finished, from the specimen list 123 into a result judgment list 401 in the result judgment window 400. An example of the display of the result judgment window 400 is illustrated in FIG. 5. Subsequently, the display controlling unit 114 displays the result judgment list 401 (step S14). After that, if there is an examination item that follows (step S15: Yes), the display controlling unit 114 returns to step S13. On the contrary, if there is no examination item that follows (step S15: No), the display controlling unit 114 ends the process. Because the culture time period for the examined bacteria varies for each of the examination items, the display controlling unit 114 may display the result judgment list 401 in the ascending order of the culture time periods of the examined bacteria.

### <A flow in the result judging process>

Next, a flow in the result judging process will be explained. In the result judgment window 400, the display controlling unit 114 displays the result judgment list 401 rearranged by the arranging unit 113. An example of the display of the result judgment window 400 is illustrated in FIG. 5. The display controlling unit 114 displays the result judgment list 401 for each of the examination items. For example, the display controlling unit 114 uses the numbers of colonies of Staphylococcus aureus, E. coli, and general viable bacteria as examination items and displays each of the examination items in a result judgment list 401. The colony examination device 100 sequentially receives judgment results from the first to the last rows displayed in the result judgment list 401. The display controlling unit 114 then changes the "judgment result" column in the result judgment list 401, based on the judgment results. In the following sections, a flow in the result judging process performed for each of the examination items will specifically be explained.

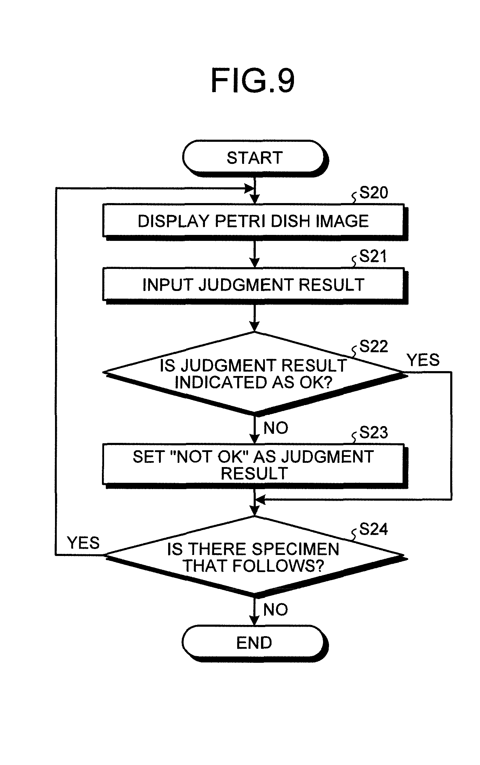

FIG. 9 is a flowchart of an example of the result judging process. First, the display controlling unit 114 displays a Petri dish image corresponding to the specimen number in the result judgment list 401, in the image display area 402 of the result judgment window 400 (step S20). Subsequently, when a judgment result is input, the colony examination device 100 receives a judgment result for the specimen arranged in the first row being displayed (step S21). When having received information indicating that the judgment result is OK (step S22: Yes), the colony examination device 100 proceeds to the process at step S24. On the contrary, when having received information indicating that the judgment result is not OK (step S22: No), the colony examination device 100 sets "not OK" as the judgment result (step S23).

Subsequently, if there is a specimen that follows at step S24 (step S24: Yes), the display controlling unit 114 returns to step S20 and displays the result judgment list 401 for the next specimen. On the contrary, there is no specimen that follows at step S24 (step S24: No), the display controlling unit 114 ends the process.

### <Advantageous effects of the first embodiment>

The colony examination device 100 includes the obtaining unit 111 that obtains, for each of the plurality of Petri dishes containing the colonies of bacteria, the piece of information used for identifying the specimen; and the identifying unit 112 that identifies each of the specimens from which the colonies of bacteria were cultured in a different one of the plurality of Petri dishes. Further, the colony examination device 100 further includes the arranging unit 113 that arranges the obtained pieces of information identifying the specimens, based on the time period it takes for the product represented by each of the specimens to arrive at the shipment delivery destination from the facility that produced the identified specimen. Also, the colony examination device 100 further includes the display controlling unit 114 that displays the arranged pieces of information identifying the specimens, in a list. By using this configuration, it is possible to appropriately set the order in which the specimens are examined or in which the images of the specimens are taken. Details of the process of setting the order in which the images of the specimens are taken will be explained later.

Further, for example, the arranging unit 113 arranges the pieces of information identifying the specimens in the ascending order of the time periods it takes for each of the products represented by the specimens to arrive at the shipment delivery destination from the facility that produced the identified specimen. With this configuration, it is possible to avoid a disadvantageous situation where a delay would occur in the sanitary examinations to be performed on neglected products, which would also cause a delay in storefront shelving of the products delivered from the facility or in secondary processing to be applied to the products delivered from the facility.

Further, the specimen number includes the Petri dish images obtained by culturing the plurality of types of bacteria for each of the specimens. Further, with respect to any of the specimens, when one of the Petri dish images obtained by culturing the plurality of types of bacteria is judged to be abnormal, the arranging unit 113 arranges the Petri dish images of the specimen obtained by culturing the other types of bacteria, so as to be in upper positions in the list.

### <Second Embodiment>

In a second embodiment, a mode in which sanitary examinations are performed on products before shipping will be explained. For example, products shipped from a facility include products having a long time period before a best-before date/time such as frozen food products. In those situations, the facility ships the products after performing the examinations thereon. A process performed by the constituent elements of the controlling unit 110 in those situations will be explained below.

From the specimen DB 121 illustrated in FIG. 3, the obtaining unit 111 obtains pieces of specimen data corresponding to specimens Nos. 111 to 114 on which sanitary examinations are to be performed. Subsequently, the identifying unit 112 identifies each of the specimens based on the specimen numbers of the pieces of specimen data obtained from the specimen DB 121.

The arranging unit 113 rearranges the pieces of specimen data obtained by the obtaining unit 111, based on the transport time periods thereof.

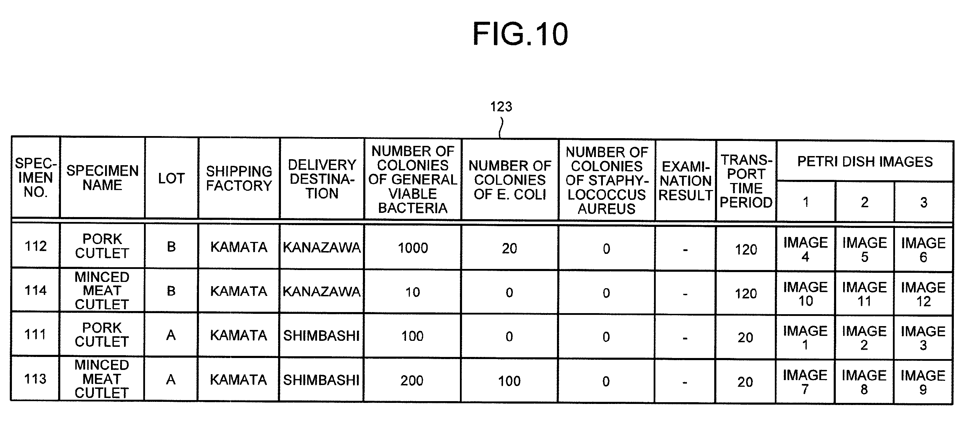

A process performed by the arranging unit 113 when the sanitary examinations are performed on products before shipping will be explained, with reference to FIG. 10. FIG. 10 is a table illustrating a second example of the specimen list created by the arranging unit 113. From among the pieces of specimen data corresponding to the specimen numbers "111", "112", "113", and "114", which are the pieces of specimen data obtained by the obtaining unit 111, the arranging unit 113 obtains the transport time periods "20", "120", "20", and "120". Subsequently, the arranging unit 113 rearranges the pieces of specimen data in descending order of the transport time periods. In other words, the arranging unit 113 rearranges the pieces of specimen data in the order of specimen numbers "112", "114", "111", and "113". In this situation, because the transport time periods of specimens Nos. "111" and "113" and the transport time periods of specimens Nos. "112" and "114" are equal to each other, the arranging unit 113 may arrange the two specimens having mutually-the-same transport time period in the opposite order in the specimen list 123.

Processes performed by the arranging unit 113 and the display controlling unit 114 will be explained by using a specific example. First, the colony examination device 100 causes the number of colonies of Staphylococcus aureus to be displayed in the "Number of colonies of Staphylococcus aureus" column of the result judgment list 401. Further, in the initial state where the result judgment list 401 created based on the specimen list 123 is displayed in the result judgment window 400, the colony examination device 100 causes "-" to be displayed in the "judgment result" column of the result judgment list 401.

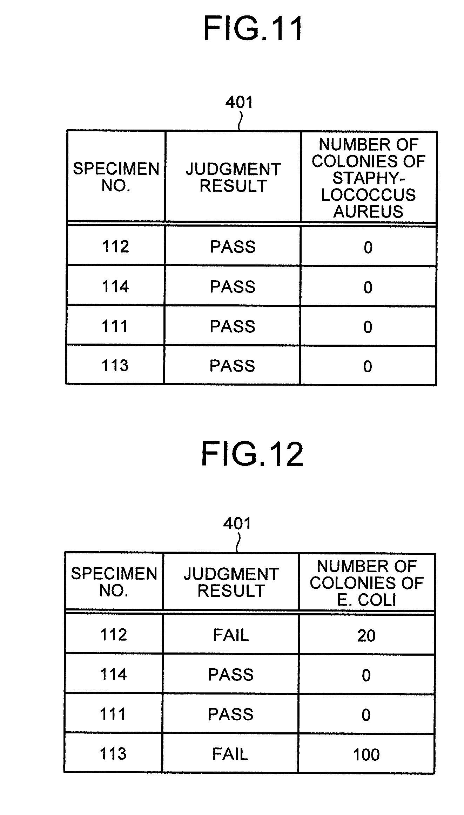

The display controlling unit 114 inputs judgment results into the "judgment result" column, according to the judgment result for each of the pieces of specimen data. In one example, the display controlling unit 114 may display the Petri dish image for the next row in the next result judgment list 401 when either the PASS button 410 or the FAIL button 411 is pressed in the result judgment window 400. Alternatively, the display controlling unit 114 may display the next Petri dish image when one of the rows in the result judgment list is clicked. FIG. 11 is a table illustrating a fourth example of the result judgment list. FIG. 11 illustrates an example of the result judgment list in which, as for the number of colonies of Staphylococcus aureus, because all the pieces of specimen data are indicated as "0", "PASSED" is input for each of all the judgment results. The display controlling unit 114 stores the judgment results into the storage unit 120 as pieces of judgment data. Further, because all the judgment results are indicated as "PASSED", the arranging unit 113 does not rearrange the pieces of specimen data.

After the examination on Staphylococcus aureus is completed, the display controlling unit 114 displays a result judgment list 401 for E. coli, based on the judgment data stored in the storage unit 120. In that situation, the colony examination device 100 causes the number of colonies of E. coli to be displayed in the "Number of colonies of E. coli" column of the result judgment list 401. Further, in the initial state where the result judgment list 401 created based on the specimen list 123 is displayed in the result judgment window 400, the colony examination device 100 causes "-" to be displayed in the "judgment result" column of the result judgment list 401. FIG. 12 is a table illustrating a fifth example of the result judgment list. In the example illustrated in FIG. 12, the colony examination device 100 causes the result judgment list 401 to be displayed based on the specimen list 123 rearranged by the arranging unit 113 according to the judgment results on the numbers of colonies of Staphylococcus aureus. In the result judgment list 401 illustrated in FIG. 12, the judgment results of specimen No. 112 and specimen No. 113 are indicated as "FAILED". The display controlling unit 114 stores the judgment results into the storage unit 120 as pieces of judgment data. Subsequently, the arranging unit 113 rearranges the data in the specimen list 123 so that specimen No. 112 is in the third row, whereas specimen No. 113 is in the fourth row.

After the examination on E. coli is completed, the display controlling unit 114 displays a result judgment list 401 for general viable bacteria, based on the judgment data stored in the storage unit 120. In that situation, the colony examination device 100 causes the number of colonies of general viable bacteria to be displayed in the "Number of colonies of general viable bacteria" column of the result judgment list 401. Further, in the initial state where the result judgment list 401 created based on the specimen list 123 is displayed in the result judgment window 400, the colony examination device 100 causes "-" to be displayed in the "judgment result" column of the result judgment list 401.

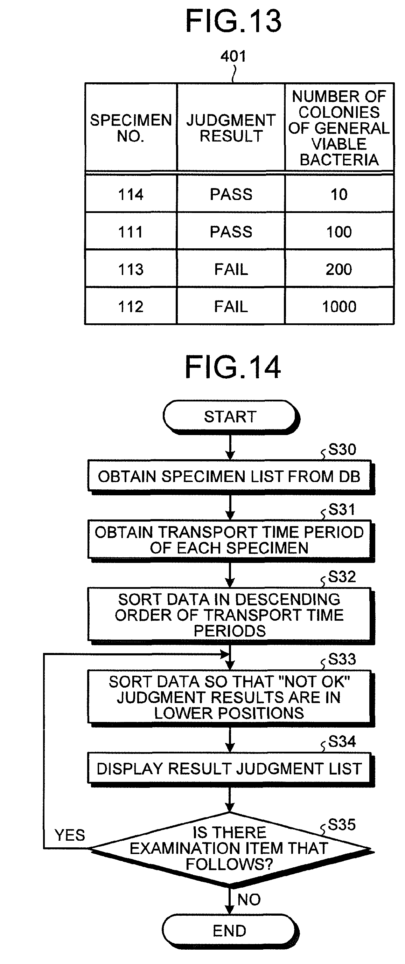

FIG. 13 is a table illustrating a sixth example of the result judgment list. In the example illustrated in FIG. 13, the colony examination device 100 causes the result judgment list 401 to be displayed based on the specimen list 123 rearranged by the arranging unit 113 according to the judgment results on the numbers of colonies of E. coli. The display controlling unit 114 displays the pieces of specimen data in the result judgment list 401 according to the order rearranged by the arranging unit 113. The display controlling unit 114 stores the judgment results into the storage unit 120 as pieces of judgment data. After that, the colony examination device 100 updates the "examination result" column of the specimen list 123, based on the pieces of judgment data for each of the examination items. For each of the examination items, if at least one of the judgment results is indicated as "FAILED", the colony examination device 100 inputs "not OK" in the "examination result" column of the specimen list 123.

### <A flow in a process performed by the colony examination device>

Next, a flow in a process performed by the colony examination device 100 will be explained. FIG. 14 is a flowchart of an example of a processing operation up to when the colony examination device displays a result judgment list. First, the obtaining unit 111 obtains one or more of the rows of the specimen DB 121 as a specimen list (step S30). Subsequently, the identifying unit 112 identifies the specimens based on the specimen numbers of the pieces of specimen data obtained from the specimen DB 121.

Subsequently, the arranging unit 113 obtains the transport time period of each of the specimens, from the pieces of specimen data (step S31). After that, the arranging unit 113 arranges the pieces of specimen data in the descending order of the transport time periods (step S32). Subsequently, the arranging unit 113 rearranges one or more pieces of specimen data of which the examination results are indicated as "not OK" into lower positions of the specimen list 123 (step S33). After that, the display controlling unit 114 extracts a part of the specimen list 123 and causes the extracted part as a result judgment list 401. Subsequently, the display controlling unit 114 displays the result judgment list 401 (step S34). After that, if there is an examination item that follows (step S35: yes), the display controlling unit 114 returns to step S33. On the contrary, if there is no examination item that follows (step S35: No), the display controlling unit 114 ends the process.

### <Advantageous effects of the second embodiment>

For example, the arranging unit 113 arranges the pieces of information identifying the Petri dishes in the descending order of the transport time periods. For example, the arranging unit 113 outputs the specimen list 123 in which the pieces of specimen data obtained by the obtaining unit 111 are rearranged in the descending order of the transport time periods. Subsequently, the display controlling unit 114 displays the result judgment lists 401 in the descending order of the culture time periods of the examined bacteria, based on the output specimen list 123. After that, the colony examination device 100 causes the Petri dish images and the like corresponding to the specimens to be displayed, according to the order in the result judgment list 401. In that situation, the colony examination device 100 receives the inputs of the judgment results on the specimens from the person in charge of the examinations. With these arrangements, because a higher priority is given to the pieces of specimen data having the longer transport time periods, it is possible to avoid a disadvantageous situation where the arrival of the products at the delivery destination would get behind schedule due to a wait for examination results and where it would become impossible to perform storefront shelving and/or secondary processing as scheduled at the delivery destination.

The specimen number includes the Petri dish images obtained by culturing the plurality of types of bacteria for each of the specimens. Further, with respect to any of the specimens, when one of the Petri dish images obtained by culturing the plurality of types of bacteria is judged to be abnormal, the arranging unit 113 arranges the Petri dish images of the specimen obtained by culturing the other types of bacteria, so as to be in lower positions in the list. With these arrangements, because the priority level of the piece of specimen data including the abnormality judgment result is lowered, it is possible to avoid a disadvantageous situation where the arrival of the products at the delivery destination would get behind schedule due to a wait for examination results and where it would become impossible to perform storefront shelving and/or secondary processing as scheduled at the delivery destination.

### <Other embodiments>

In the first and the second embodiments, the example is explained in which the storage unit 120 stores the specimen list 123 therein; however, possible embodiments are not limited to this example. The specimen list 123 may be stored in a portable recording medium such as a Compact Disk Read-Only Memory (CD-ROM), a Digital Versatile Disk (DVD), or a Universal Serial Bus (USB) memory, or a semiconductor memory such as a flash memory, or an external storage device.

Further, the number of colonies in each of the Petri dishes may visually be counted by an examiner or may automatically be counted by employing a device such as a colony counter.

In the first and the second embodiments, the example is explained in which the arranging unit 113 rearranges the data in accordance with the transport time periods and further rearranges the data according to the judgment results for each of the examination items. However, possible embodiments are not limited to this example. After rearranging the data in accordance with the transport time periods, the arranging unit 113 does not necessarily have to rearrange the data according to the judgment results for each of the examination items.

The display controlling unit 114 may display the Petri dish image for the next row in the next result judgment list 401, when either the PASS button 410 or the FAIL button 411 is pressed in the result judgment window 400. Alternatively, the display controlling unit 114 may display the next Petri dish image when one of the rows in the result judgment list is clicked.

### <The order in which the images of the Petri dishes are taken>

In the first embodiment, the example is explained in which the examiner takes the images of the Petri dishes by referring to the specimen numbers displayed on the monitor of a device imaging the Petri dishes and setting the Petri dish corresponding to each of the specimen numbers on the device. However, the order in which the images of the Petri dishes are taken may be set in the manner described below.

First, the examiner collects a part of each of the food items as a specimen and assigns a specimen number to the specimen. Subsequently, when the examination items include examinations for the numbers of colonies of general viable bacteria, E. coli, and Staphylococcus aureus, the examiner divides the collected specimen into three sections and put each of the sections into a Petri dish. After that, the examiner allows the specimens in the Petri dishes to be cultured, by keeping the Petri dishes for one to two days at a temperature suitable for growth of the bacteria.

Further, the colony examination device 100 rearranges the order in which images of the specimens cultured by the examiner are to be taken, based on the transport time periods of the food items. More specifically, at first, with respect to the specimen list 123 obtained from the examination DB 121, the arranging unit 113 rearranges the pieces of data in the specimen list 123 in accordance with the transport time periods. For example, the arranging unit 113 may arrange the pieces of data in ascending order of the transport time periods, similarly to the example in the first embodiment. Alternatively, the arranging unit 113 may arrange the pieces of data in descending order of the transport time periods, similarly to the example in the second embodiment. Subsequently, the arranging unit 113 causes the monitor of the device imaging the Petri dishes to display the pieces of specimen data in the rearranged order.

Further, because the monitor of the device imaging the Petri dishes displays a specimen number, the examiner sets the Petri dish corresponding to the specimen number on the device and causes the image of the specimen to be taken. After finishing taking the image of the Petri dish, the colony examination device 100 causes the next piece of specimen data to be displayed. After that, when all the examinations are completed, the colony examination device 100 performs an examination on each of the specimens according to the order in which the images of the specimens were taken. With these arrangements, it is possible to avoid a disadvantageous situation where, when the images of the specimens are taken according to the order in which the specimens are arranged in the list, the sanitary examinations would be performed according to the order in which the image taking processes of the specimens are finished.

### <When there are multiple values of transport time periods with respect to a single lot>

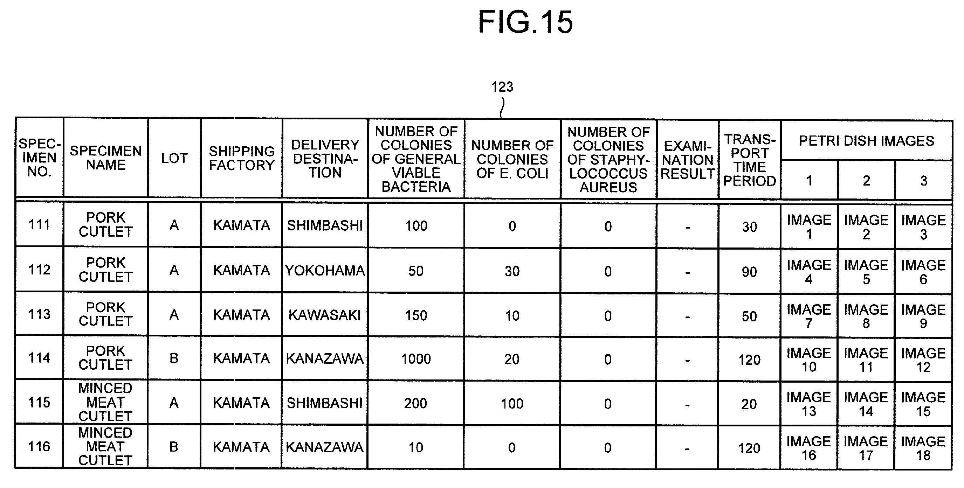

As explained above, each of the "lot" symbols in the examination DB 121 is uniquely assigned to products to be examined that the same as one another. Thus, even if the delivery destinations are mutually different, when the products to be examined are the same as one another, the same lot symbol may be assigned. FIG. 15 is a table illustrating a specimen list corresponding to when there are multiple values of transport time periods with respect to a single lot. Although the pieces of specimen data corresponding to the specimen numbers 111 to 113 have mutually-the-same specimen name, mutually-the-same specimen lot symbol is assigned thereto.

When the products are to be shipped by using the mode in which the sanitary examinations are performed on the products after shipping, the arranging unit 113 rearranges the pieces of specimen data, by using such a piece of specimen data that has the shortest transport time period, among the pieces of specimen data to which mutually-the-same lot symbol is assigned.

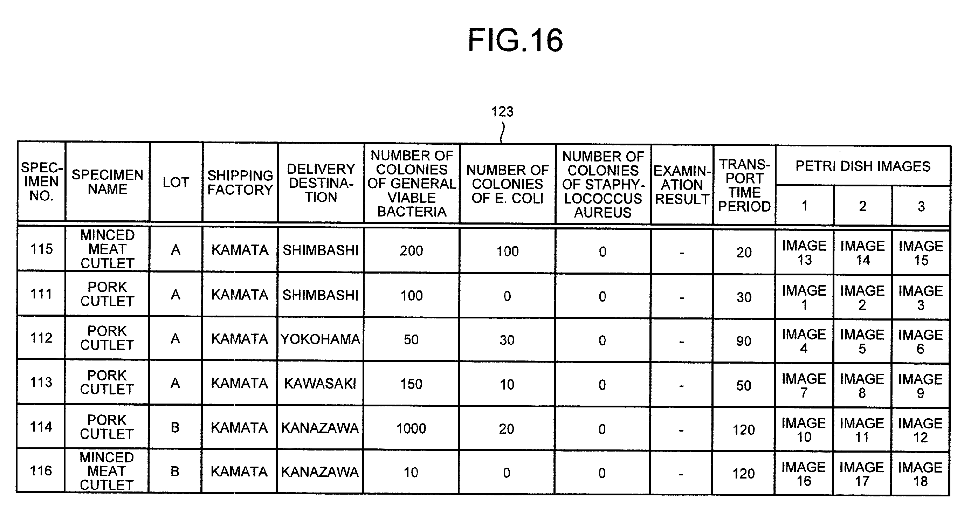

FIG. 16 is a table illustrating a first example of a specimen list rearranged by the arranging unit when there are multiple values of transport time periods with respect to a single lot. The arranging unit 113 rearranges the pieces of specimen data, by using the transport time period of specimen No. 111 having the shortest transport time period "30", among the pieces of specimen data corresponding to the specimens numbers 111 to 113. As illustrated in FIG. 16, the arranging unit 113 arranges the pieces of specimen data corresponding to the specimen numbers 111 to 113, to be positioned below specimen No. 115 of which the transport time period is "20".

In contrast, when the products are to be shipped by using the mode in which the sanitary examinations are performed on the products after shipping, the arranging unit 113 rearranges the pieces of specimen data by using such a piece of specimen data that has the longest transport time period, among the pieces of specimen data to which mutually-the-same lot symbol is assigned.

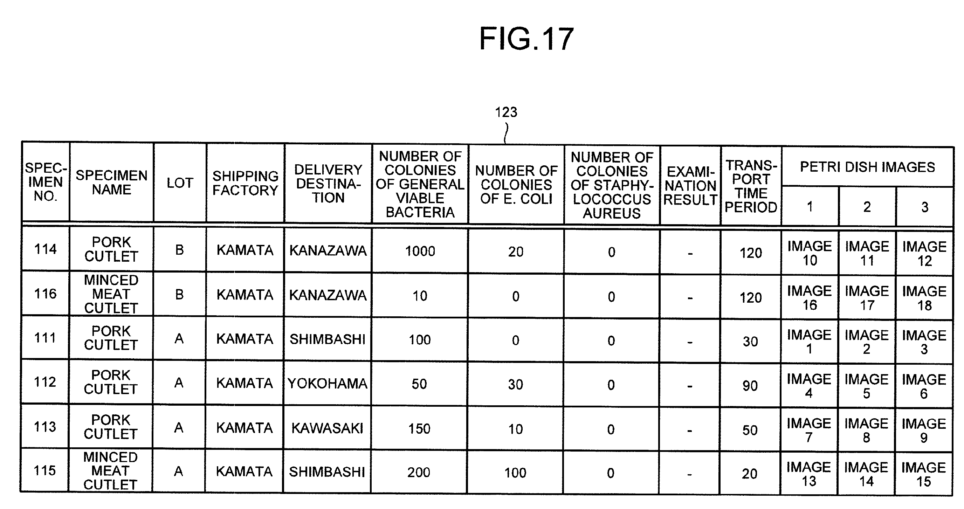

FIG. 17 is a table illustrating a second example of the specimen list rearranged by the arranging unit when there are multiple values of transport time periods with respect to a single lot. The arranging unit 113 rearranges the pieces of specimen data by using the transport time period of specimen No. 112 that has the longest transport time period "90", among the pieces of specimen data corresponding to the specimen numbers 111 to 113. As illustrated in FIG. 17, the arranging unit 113 arranges the pieces of specimen data corresponding to the specimen numbers 111 to 113 so as to be positioned below specimen No. 116 of which the transport time period is "120".

As explained above, as a result of the arranging unit rearranging the pieces of specimen data, it is possible to improve the efficiency in the examinations by putting together the pieces of specimen data belonging to mutually-the-same lot, because mutually-the-same lot tends to have mutually-the-same examination result.

### <An arranging process that uses best-before dates/times or expiration dates/times>

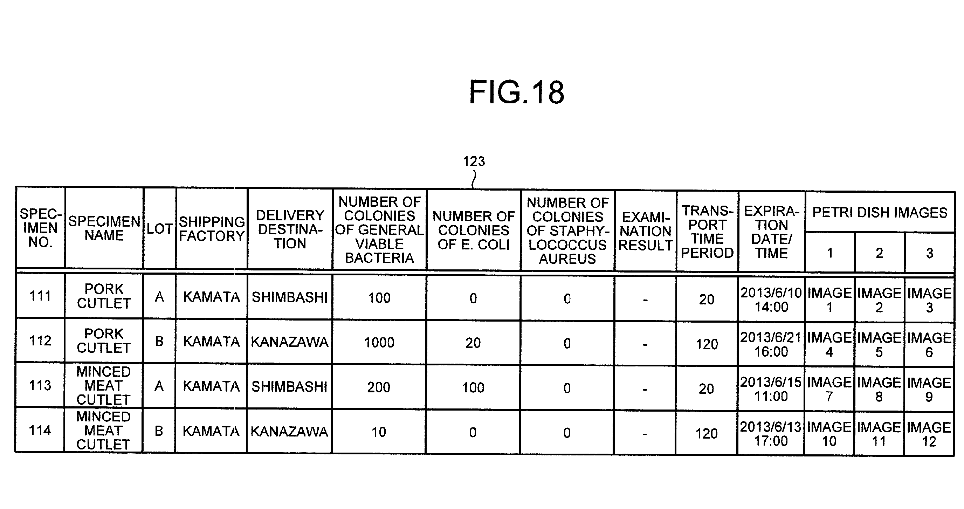

When there are two or more pieces of specimen data of which the transport time periods are the same as one another and of which the best-before dates/times or the expiration dates/times are different from one another, the arranging unit 113 may arrange the pieces of specimen data in ascending order of time periods until the best-before dates/times or the expiration dates/times. FIG. 18 is a table illustrating an example of a specimen list containing best-before dates/times as items thereof. Specimens Nos. 111 and 113 have mutually-the-same transport time period "20". Further, specimens Nos. 112 and 114 have mutually-the-same transport time period "120".

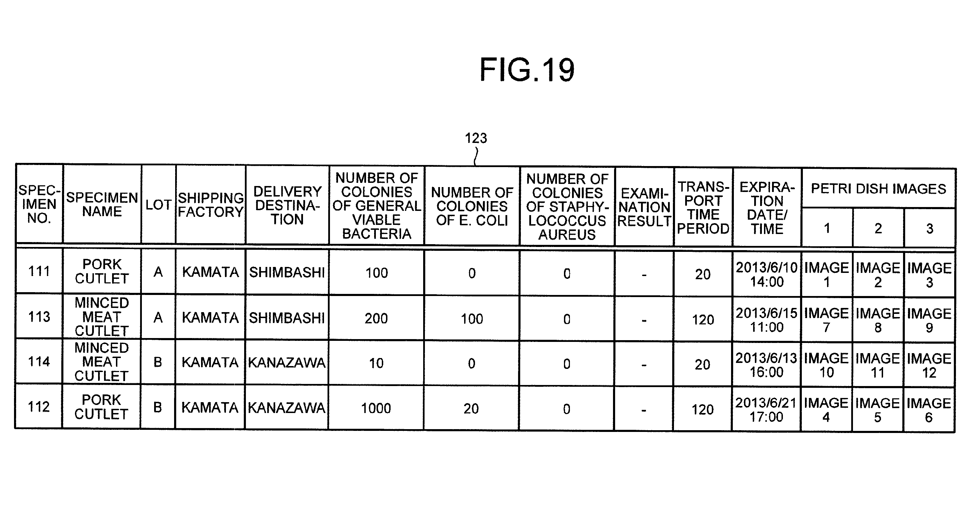

FIG. 19 is a table illustrating an example of a specimen list containing expiration dates/times as items thereof and having been rearranged by the arranging unit. In this situation, the arranging unit 113 arranges, in the first row, the piece of specimen data corresponding to specimen No. 111 of which the transport time period is "20" and of which the time period until the expiration date/time is shorter than that of specimen No. 113. Subsequently, the arranging unit 113 arranges, in the second row, the piece of specimen data corresponding to specimen No. 113 of which the transport time period is "20". After that, the arranging unit 113 arranges, in the third row, the piece of specimen data corresponding to specimen No. 114 of which the transport time period is "120" and of which the time period until the expiration date/time is shorter than that of specimen No. 112. Subsequently, the arranging unit 113 arranges, in the fourth row, the piece of specimen data corresponding to specimen No. 112 of which the transport time period is "120". With these arrangements, it is possible to transport the products, while giving a higher priority to specimens having a shorter time period before the expiration dates/times thereof. Also, when the specimen list 123 includes best-before dates/times, the arranging unit 113 arranges the pieces of data in the specimen list 123 in the same manner as described above.

### <A hardware configuration of a display terminal>

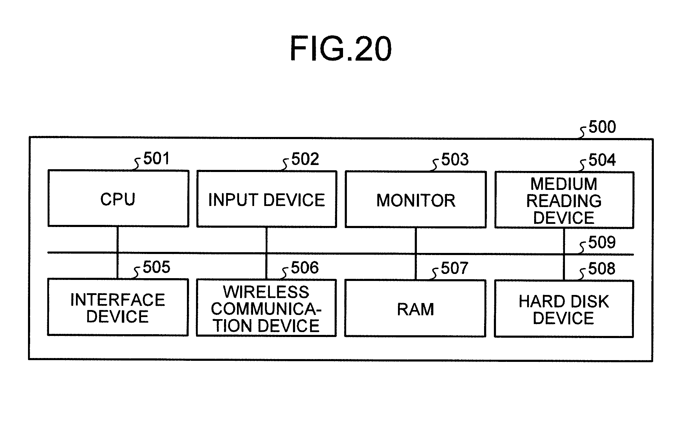

FIG. 20 is a diagram illustrating a hardware configuration of a computer related to a colony examination device. As illustrated in FIG. 20, a computer 500 includes: a CPU 501 that performs various types of computation processes; an input device 502 that receives data inputs from a user; and a monitor 503. Further, the computer 500 also includes: a medium reading device 504 that reads a computer program (hereinafter, "program") or the like from a storage medium; an interface device 505 that establishes a connection to another apparatus; and a wireless communication device 506 that establishes a wireless connection to another apparatus. Further, the computer 500 also includes: a RAM 507 that temporarily stores therein various types of information; and a hard disk device 508. Further, the devices 501 to 508 are connected to a bus 509.

The hard disk device 508 has stored therein a colony examination computer program (hereinafter, "colony examination program") that has the same functions as those of the processing units such as the obtaining unit 111, the identifying unit 112, the arranging unit 113, and the display controlling unit 114 included in the controlling unit 110 illustrated in FIG. 2. Further, the hard disk device 508 has stored therein various types of data used for realizing the colony examination program.

The CPU 501 performs various types of processes by reading the programs stored in the hard disk device 508, loading the read programs into the RAM 507, and executing the loaded programs. Further, these programs are capable of causing the computer 500 to function as the obtaining unit 111, the identifying unit 112, the arranging unit 113, and the display controlling unit 114 illustrated in FIG. 2.

The colony examination program described above does not necessarily have to be stored in the hard disk device 508. For example, another arrangement is acceptable in which the program stored in a storage medium that is readable by the computer 500 is read and executed by the computer 500. Examples of the storage medium that is readable by the computer 500 include a portable recording medium such as a CD-ROM, a DVD, or a USB memory, a semiconductor memory such as a flash memory, and a hard disk drive. Further, yet another arrangement is also acceptable in which the program is stored in an apparatus connected to a public communication line, the Internet, a Local Area Network (LAN) or the like, so that the computer 500 is able to read the program through any of these and to execute the program.

### [Explanation of Reference]

- 100: COLONY EXAMINATION DEVICE

- 101: INTERFACE (I/F)
- 102: DISPLAY UNIT
- 103: OUTPUT UNIT
- 110: CONTROLLING UNIT
- 111: OBTAINING UNIT
- 112: IDENTIFYING UNIT
- 113: ARRANGING UNIT
- 114: DISPLAY CONTROLLING UNIT
- 120: STORAGE UNIT
- 121: EXAMINATION DATABASE (DB)
- 122: DISPLAY DATA
- 123: SPECIMEN LIST

## Claims

1. A colony examination computer program that causes a computer to execute a process comprising:
obtaining, for each of a plurality of specimens from which colonies of bacteria are cultured, a piece of information used for identifying the specimen;
arranging the pieces of information identifying the specimens, based on a time period a product represented by each of the specimens takes to arrive at a shipment delivery destination from a facility that produced the specimen, the time period being indicated in each of the obtained pieces of information identifying the specimens; and
displaying the arranged pieces of information identifying the specimens, in a list.

2. The colony examination computer program according to claim 1, wherein the arranging includes arranging the pieces of information identifying the specimens in ascending order of the time periods.

3. The colony examination computer program according to claim 1, wherein the arranging includes arranging the pieces of information identifying the specimens in descending order of the time periods.

4. The colony examination computer program according to claim 1, wherein, when pieces of information identifying specimens having a mutually-same time period are to be arranged, the arranging includes arranging the pieces of information identifying the specimens in ascending order of time periods until best-before dates/times or expiration dates/times of the products represented by the specimens.

5. The colony examination computer program according to claim 1, wherein
each of the pieces of information identifying the specimens is kept in correspondence with images of Petri dishes in each of which a different one of a plurality of types of bacteria has been cultured with respect to the single specimen; and
when one of the images of the Petri dishes in each of which a different one of the plurality of types of bacteria has been cultured with respect to any one of the specimens is judged to be abnormal, the arranging includes arranging one or more pieces of information identifying the specimen from which one or more other types of bacteria among the types of bacteria have been cultured with respect to the specimen, so as to be in one or more upper or lower positions in the list.

6. A colony examination device comprising:
an obtaining unit configured to obtain, for each of a plurality of specimens from which colonies of bacteria are cultured, a piece of information used for identifying the specimen;
an arranging unit configured to arrange the pieces of information identifying the specimens, based on a time period a product represented by each of the specimens takes to arrive at a shipment delivery destination from a facility that produced the specimen, the time period being indicated in each of the pieces of information identifying the specimens and having been obtained by the obtaining unit; and
a display controlling unit configured to display the pieces of information identifying the specimens and having been arranged by the arranging unit, in a list.

7. The colony examination device according to claim 6, wherein the arranging unit is configured to arrange the pieces of information identifying the specimens in ascending order of the time periods.

8. The colony examination device according to claim 6, wherein the arranging unit is configured to arrange the pieces of information identifying the specimens in descending order of the time periods.

9. The colony examination device according to claim 6, wherein, when pieces of information identifying specimens having a mutually-same time period are to be arranged, the arranging unit is configured to arrange the pieces of information identifying the specimens in ascending order of time periods until best-before dates/times or expiration dates/times of the products represented by the specimens.

10. The colony examination device according to claim 6, wherein
each of the pieces of information identifying the specimens is kept in correspondence with images of Petri dishes in each of which a different one of a plurality of types of bacteria has been cultured with respect to the single specimen; and
when one of the images of the Petri dishes in each of which a different one of the plurality of types of bacteria has been cultured with respect to any one of the specimens is judged to be abnormal, the arranging unit is configured to arrange one or more pieces of information identifying the specimen from which one or more other types of bacteria among the types of bacteria have been cultured with respect to the specimen, so as to be in one or more upper or lower positions in the list.

11. A colony examination method by which a computer executes:
obtaining, for each of a plurality of specimens from which colonies of bacteria are cultured, a piece of information used for identifying the specimen;
arranging the pieces of information identifying the specimens, based on a time period a product represented by each of the specimens takes to arrive at a shipment delivery destination from a facility that produced the specimen, the time period being indicated in each of the obtained pieces of information identifying the specimens; and
displaying the arranged pieces of information identifying the specimens, in a list.

12. The colony examination method according to claim 11, wherein the arranging includes arranging the pieces of information identifying the specimens in ascending order of the time periods.

13. The colony examination method according to claim 11, wherein the arranging includes arranging the pieces of information identifying the specimens in descending order of the time periods.

14. The colony examination method according to claim 11, wherein, when pieces of information identifying specimens having a mutually-same time period are to be arranged, the arranging includes arranging the pieces of information identifying the specimens in ascending order of time periods until best-before dates/times or expiration dates/times of the products represented by the specimens.

15. The colony examination method according to claim 11, wherein
each of the pieces of information identifying the specimens is kept in correspondence with images of Petri dishes in each of which a different one of a plurality of types of bacteria has been cultured with respect to the single specimen; and
when one of the images of the Petri dishes in each of which a different one of the plurality of types of bacteria has been cultured with respect to any one of the specimens is judged to be abnormal, the arranging includes arranging one or more pieces of information identifying the specimen from which one or more other types of bacteria among the types of bacteria have been cultured with respect to the specimen, so as to be in one or more upper or lower positions in the list.
